(19) Europäisches Patentamt
European Patent Office
Office européen des brevets

(11) Publication number: **0 236 271 B1**

(12) **EUROPEAN PATENT SPECIFICATION**

(45) Date of publication of patent specification: **21.08.91**

(51) Int. Cl.5: **A61K 9/68**

(21) Application number: **87810104.7**

(22) Date of filing: **23.02.87**

# (54) Antacid chewing gum.

(30) Priority: **27.02.86 US 834301**

(43) Date of publication of application:
**09.09.87 Bulletin 87/37**

(45) Publication of the grant of the patent:
**21.08.91 Bulletin 91/34**

(84) Designated Contracting States:
**BE CH DE ES FR GB GR LI SE**

(56) References cited:
**EP-A- 0 082 670**
**EP-A- 0 122 808**
**EP-A- 0 166 440**
**EP-A- 0 176 237**
**GB-A- 934 596**
**GB-A- 2 084 845**
**US-A- 4 238 475**
**US-A- 4 446 135**

(73) Proprietor: **WARNER-LAMBERT COMPANY**
**201 Tabor Road**
**Morris Plains New Jersey 07950(US)**

(72) Inventor: **Cherukuri, Subraman Rao**
**10 Jean Drive**
**Towaco, N.J. 07082(US)**
Inventor: **Calabro, Frank P.**
**3R Village Green**
**Budd Lake, N.J. 07828(US)**
Inventor: **Mansukhani, Gul**
**10 Travis Avenue**
**Staten Island, N.Y. 10314(US)**
Inventor: **Rieger, Martin M.**
**304 Mountain Way**
**Norris Plains, N.J. 07950-1910(US)**
Inventor: **Elefant, Milton**
**22 Mohawk Drive**
**Livingston, N.J. 07039(US)**

(74) Representative: **Jones, Michael Raymond et al**
**HASELTINE LAKE & CO. Hazlitt House 28**
**Southampton Buildings Chancery Lane**
**London WC2A 1AT(GB)**

**EP 0 236 271 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention concerns a medicament containing non-staling chewing gum and particularly chewing gun useful for delivery of measured doses of antacids.

Attempts have been made in the past to utilize gum as a carrier for medicaments. Chewing gum is relatively inexpensive, its manufacturing processes are well known and gum can be flavoured to minimize the negative taste sensations associated with certain medicaments.

U.S. Patent 4,265,877 issued to Louis T. Tenta discloses a chewing gum base with a mixture of sodium fluoride and calcium carbonate in the form of oyster shell distributed within the base. This invention is directed toward the use of that combination to introduce fluoride as a suitable medicament. Calcium carbonate is routinely used as a filler for chewing gum, and calcium carbonate as well as other water insoluble salts remain within the gum base during mastication.

GB 934596 which corresponds to U.S. Patent 3,011,949 discloses the concept of spray drying otherwise insoluble particulate medicaments, such as calcium phosphate, antacids, etc., with sugar and mixing with sugar in a conventional gum formulation. According to this patent, mastication liberates a large percentage of the medicament as well as the sugar that was spray dried with it. Subsequent experiments have, however, failed to duplicate the high degree of insoluble medicament liberation set forth in this patent. Apparently, the degree of liberation is highly dependent upon the particular spray drying process used.

U.S. Patent 4,208,432 to Ker-Tchi Noborio discloses the use of either alpha lactose, beta lactose or calcium carbonate along with certain saturated fatty acid monoglycerides as a powdery releasing agent.

EP-A-0082670 which relates to the problem of producing a non-staling chewing gum composition having a low moisture content discloses a composition containing less than 2% by weight water and having 8 to 18% by weight glycerin. The release of medicaments from this composition is not considered in this document.

Frank Witzell et al in U.S. Patent 4,238,475 discloses introducing magnesium hydroxide or calcium carbonate or the like in chewing gum by including a water soluble phase formed of an aqueous softener with the softener being coated by a water soluble coating agent. This sequence of steps is part of an otherwise conventional process for manufacture of chewing gum.

Copending EP-A-0176237, which was published after the priority date of this application, discloses a non-staling low moisture chewing gum with substantial organoleptic and stability advantages as well as process advantages when compared to conventional chewing gum. The chewing gum composition disclosed has a moisture content of up to 1.0% by weight of the final composition and comprises a gum base which softens in a temperature range of about 40° to about 60°C, a flavoring agent, softener such as lecithin and the like and a sweetening agent. The ingredients contain only residual moisture and are added to the composition without the use of aqueous solutions.

It is essential that the final chewing gum composition must have a moisture content which is in equilibrum, ie. remains substantially constant at ambient relative humidity. The relative humidity value at which the water content is at equilibrium is a means for identifying the susceptibility of a composition to gain or lose moisture which in turn relates to the tendency for the gum to remain moisture stable, i.e., not to dry out or become stale. When the gum base disclosed in EP-A-0176237 neither picks up nor loses moisture it is in a state of equilibrium with the environment. The relative humidity at which the moisture content is in equilibrium depends on the ratio of free moisture to bound moisture in a product as well as The temperature of the product and environment. The amount and rate at which a chewing gum losses or gains moisture depends upon the differential between the relative humidity at which the products' water content is at equillibrium and the ambient relative humidity. Since the relative humidity at which the moisture content of the gum disclosed in EP-A-0176237 is at equillibrium is substantially below that of the environment in most geographic regions, the gum disclosed in EP-A-0176237 will not lose moisture. The relative humidity at which the moisture content is at equillibrium of the gum disclosed is between 15 and 30% and generally between 21 and 25% at about 23°C to retain this differential.

The factors, according to EP-A-0176237, which provide the extended shelf life associated with the low moisture gum are first, omitting moisture and moisture containing ingredients in the chewing gum formulations; second, maintaining the relative humidity at which the moisture content of the chewing gum composition is at equilibrium at a lower level than the ambient relative humidity; and third, using a gum base which softens between the temperature of about 40°C and 60°C. The later restriction, of course, limits the use of the elastomers to produce this gum.

Another distinction claimed for the gum of EPA-0176 237 is that during processing the gum base is softened rather than melted. Softened is defined as heating to a semi-viscous state in which the viscosity is relatively high and the base has better film forming and stretching characteristics than bases which are

melted. The preferred softening temperature is between 50 and 55°.

The amount of base varies between 5 and 55% by weight of the final gum compositions with 20 to 35% by weight being preferred.

The gum disclosed in EP-A-0176237 can also utilize plasticizers or softeners such as lecithin and the like which may be incorporated in the gum base and these additional materials may be present in amounts up to about 30% by weight but preferably from 3 to about 7% by weight of the final gum base composition. Mixtures of these ingredients can also be used.

According to the present invention there is provided a medicament-containing chewing gum composition having improved delivery properties and a moisture content up to 0.3% by weight of the final composition which composition comprises:

(i) a gum base, which softens in the temperature range of 40° C to 60° C;

(ii) a softener; and

(iii) a co-spray dried mixture of a sweetening agent and a water-insoluble, particulate medicament, and wherein the moisture content of the chewing gum composition is at equilibrium in a relative humidity in the range 15 to 30% at a temperature of 23° C.

Surprisingly the use of a co-spray dried medicament in conjunction with a low moisture gum results in an extremely efficient medicament release system.

The present invention is as defined in the claims. It is further described as follows.

The chewing gum compositions of the instant invention have a moisture content of up to approximately 0.3% by weight of the final composition and comprise a gum base which softens in a temperature range of 40° to 60° C, optionally a flavouring agent, a softener such as lecithin, and a sweetening agent, with the ingredients containing only residual moisture and being added to the composition without additional moisture, i.e. without the use of aqueous solutions. The final chewing gum composition must have a water content which is at equilibrium at a relative humidity value which is lower than that of the ambient relative humidity.

There are several essential features of the anhydrous gum of the present invention. One such feature is the requirement of a soft gum base, e.g., one which softens in the temperature range disclosed above. Traditionally, gum bases were employed which melted in a range of 70°-120° C. The gum bases of the present invention are well known in the art. For the preparation of the gum of the present invention they must not be heated beyond the softening point of 60° C. Thus, while various combinations of gum bases containing high levels of polyvinyl acetate may be used, the particular combination employed must not be such that it softens above 60° C.

Another essential feature is the total moisture content and the equilibrium relative humidity value at which that moisture content of the chewing gum composition is at equilibrium. Moisture related product degradation is one of the prime stability concerns for chewing gum compositions and products. The environmental factor influencing moisture loss or gain is relative humidity. It is commonly accepted that the lower the relative humidity, the faster things dry out. Relative humidity (RH) is a measure of the vapor pressure exerted by the moisture in the atmosphere. As relative humidity increases or decreases, the pressure of the moisture in the atmosphere increases or decreases accordingly. Pure water exerts a moisture vapor pressure equivalent to 100% RH. As such, water will evaporate when stored in any environment less than 100% RH. If impurities are added to that water, the moisture vapor pressure will decrease.

Measurements of the relative humidity values at which the water content was at equilibrium, which are referred to herein as Equilibrium relative humidity (ERH) measurements, were taken for the gum compositions. ERH is a means of identifying the susceptibility or propensity of the composition to moisture gain or loss, which in turn relates to the tendency for the gum to remain moisture-stable and to not dry out or become stale. When the product neither picks up nor loses moisture, it is in a state of equilibrium with the environment. The ERH measurement depends on the ratio of free moisture to bound moisture in a product and the temperature. The amount and rate at which a chewing gum loses or gains moisture depends on the differential between the product's ERH and ambient RH. The transfer of moisture will be in the direction from high to low RH until an equilibrium state is reached.

The chewing gum compositions of the present invention have a low ERH and therefore tend not to lose moisture, since most environments have a higher RH than the compositions ERH. For example, the RH of most geographic regions is between 35-45% depending on the time of year. If the ERH of the chewing gum is greater than the ambient relative humidity, the gum will lose moisture and dry out. The ERH range of the inventive compositions, however, are between 15% to 30%, and preferably 21 to 25%$ at room temperature, e.g., approximately 23° C. Thus, there is no tendency for chewing gum made from the inventive compositions to dry out. Rather, the tendency, if any, would be to pick up moisture during shelf life. Too

much moisture pick up is undesirable however, since it causes wetting of the gum, loss of sugar coating and water solubles and ultimately sticking of the wrapper to the gum. Thus, a delicate moisture balance must be maintained whereby the product's ERH is maintained at a low value relative to the ambient relative humidity; and the total moisture content kept at a maximum of 0.3% by weight. The advantages of the chewing gum compositions of the present invention of long shelf life and freshness stability, is believed to be due to the essential features of:

a) controlling the moisture content and the amount of moisture containing ingredients in the chewing gum formulations;

b) maintaining the ERH of the chewing gum composition at a lower level than that of the ambient relative humidity; and

c) using a gum base which softens between the temperatures of 40° C and 60° C.

Those elastomers useful in the soft gum bases of the present invention include, but are not limited to, isobutylene-isoprene copolymers, polyvinylacetate, polyisobutylene, polyvinylalcohol, SBR, natural rubbers such as chicle, jelutong, balata, crown gum, gutta-percha, lechi-caspi, sorva and mixtures thereof. When using a combination of elastomers, the total elastomer mixture must be capable of being softened within the range of 40° C to 60° C, preferably 45° to 57° C, and most preferably 50° to 55° C. The process of softening is meant to be distinct from the traditional melting of gum bases. By the term "softened" is meant the gum base is heated to a semiviscous state, wherein the viscosity is relatively high and the base has better film forming and stretching characteristics than bases which are melted. Additionally, the lower temperature used to soften the base does not melt the sugar and other materials added to the gum base and as such there is believed to be little chemical interaction between the base and these materials. The conventional bases on the other hand, are heated to higher temperatures to cause the gum base to liquefy (melt). The melted base has a much lower viscosity than the inventive softened bases and as such has less of a film forming, stretching quality. Additionally, sugars and dissolvable materials also melt along with the elastomers. These materials often recrystallize out later on, making the product brittle.

The amount of gum base employed will vary greatly depending on various factors such as the type of base used, consistency desired and other components used to make the final product. In general, amounts of 5% to 55% by weight of the final chewing gum composition are acceptable for use, with preferred amounts of 15 to 40% and more preferably 20% to 35% by weight being suitable.

The gum base composition may contain elastomer solvents to aid in softening the rubber component. It is important, however, that these components be substantially free from water, since the final composition is to have a maximum moisture content up to only 0.3% by weight. Such elastomer solvents may comprise the methyl, glycerol or pentaerythritol esters of rosins or modified rosins, such as hydrogenated, dimerized or polymerized rosins or mixtures thereof. Examples of elastomer solvents suitable for use herein include pentaerythritol ester of partially hydrogenated wood rosin, pentaerythritol ester of wood rosin, glycerol ester of partially dimerized rosin, glycerol ester of polymerized rosin, glycerol ester of tall oil rosin, glycerol ester of wood rosin and partially hydrogenated wood rosin, and partially hydrogenated methyl ester of rosin and mixtures thereof. The solvent may be employed in an amount ranging from 10% to 75% and preferably 45% to 70% by weight of the gum base.

A variety of traditional ingredients used as plasticizers or softeners, such as lecithin, may be incorporated into the gum base as a softener to obtain a variety of desirable textures and consistency properties. These materials are generally employed in amounts of up to approximately 30% by weight and preferably in amounts of from 3% to 7% by weight of the final gum base composition. Mixtures of such ingredients can be used.

It is particularly preferred that the gum base be rendered increasingly hydrophilic when compared to conventional gum bases, to aid in liberating the medicament. A particularly preferred means to accomplish this is to utilize highly emulsified polyvinyl acetate as a gum base component.

The chewing gum compositions of the present invention preferably contain an edible food material which is capable of being formed into particles having microporous channels. The particles, which are preferably spherical, have preferred low bulk densities in the range of 48 to 128 kg/$m^3$ (3.0 to 8 lb./$ft.^3$) and preferably 48 to 96 kg/$m^3$ (3.0 to 6.0 lb/$ft.^3$). Materials, not having low bulk densities, coupled with microporous channels have been found less desirable for use in the inventive formulations. Such materials have been found to more quickly release the flavouring from the formulation and fail to sustain flavour-sweetness duration.

The optionally included particles referred to in the preceding paragraph may be produced from a wide range of materials. Without being limited thereto, illustrative materials are carbohydrates such as the dextrins, starch, pectin, algin, methyl cellulose, carboxy methyl cellulose, carboxy methyl amylose, carboxy methylamylopectin, dextrose, fructose, maltose, lactose, dextrins, natural gums and mixtures thereof.

Exemplary natural gums include tragacanth, acacia, arabic, locust bean, caraya, and carragean. Although the particles are not essential to the gum of the present invention, it is preferred that they be present as a means of increasing the juiciness of the chewing gum. Due to the fine, porous nature of the particles, they immediately absorb moisture from saliva when the chewing gum product is masticated. The particles swell and impart a juiciness to the gum.

Such materials while not useful for co-spray drying and medicament addition, are commercially available and may be prepared by spray drying previously expanded particles in a heated zone. For illustrative purposes, however, a preferred process for preparing the particles is described in U.S. Patent 4,180,593 to Cohan. Briefly the process disclosed therein involves spraying a flowable composition in the presence of a blowing agent, such as ammonium bicarbonate, to form beads, subjecting the beads to a heated zone to expand the beads by expansion of gases within the interior of the beads, and cooling the resulting expanded beads to stop further expansion and aid in control of bulk density.

The optionally-included particles are employed in the chewing gum composition in amounts of 0.1% to 12% by weight and preferably 0.5% to 6% by weight based on the weight of the final formulation. Amounts less than 0.1% fail to achieve enhanced flavour and sweetness perception whereas amounts higher than 12% do not achieve a pleasing flavour sweetness release.

The preferred spherical particle for use with this invention is a maltodextrin. This maltodextrin is distinct from known maltodextrins which have distinct particle sizes and are void of a microporous channel structure. Such conventional maltodextrins or corn syrup solids as they are commonly referred to, have bulk densitites from 240 to 737 kg/m$^3$ (15 to 46 lb./ft.$^3$) and D.E. values from 7 to 38. Such materials are unsuitable for use in the present invention as the microporous particles, but may be used in the instant formulations in addition to the spherical microporous particles described above. When used in this manner, they may be used in conventional amounts well known to the skilled inventor.

According to the present invention there is further provided a process for the preparation of a chewing gum according to the present invention, which process comprises the steps of:

(a) softening the gum base using a temperature in the range of 40°C to 60°C with the addition of a softener adding a co-spray dried mixture of

(b) a sweeting agent and a medicament;

(c) adding optional ingredients such as a flavouring agent or a texturizing agent, e.g., glycol; continuing to combine until a homogenous, pliable composition is obtained;

(d) extruding the composition;

(e) forming the composition into chewing gum pieces without cooling; and wrapping without prior conditioning the chewing gum.

The gum bases used in the present invention, due to their softening characteristics between the ranges disclosed above, can be made in most chewing gum mixing kettles without the special requirements of traditional gum base kettles.

Whereas the prior art process required masticating and melting of the gum base in a two-step starting batch/finished batch process usually requiring 5-6 hours, the process of the present invention requires only softening of the gum base in a one-step process taking 1-1 1/2 hours. It is significant that the inventive process saves considerable time and energy over the prior art process since this saving can be reflected in significant cost savings, more efficient production, as well as a higher quality chewing gum.

Conventional prior art processes teach melting the gum base, mixing in other chewing gum composition ingredients, and cooling the mixture prior to extrusion. Additionally, prior to wrapping, the prior art compositions are conditioned for 24-48 hours.

The inventive process, however, does not require cooling prior to extrusion nor does it require conditioning prior to wrapping. This process can be modified by softening the base directly in the gum kettle, followed by extruding, rolling and scoring and wrapping. Additionally, the gum base can be first softened by other means such as in an oven, then placed in the gum kettle.

The gum base formulation must have a softening range of between 40°C and 60°C to be useful in the process of the present invention. Additionally, to be able to carry out the process as described, no additional moisture is added. The only moisture present is residual moisture, most of which is believed to be bound moisture, inherent in certain ingredients. The total residual moisture must not yield a chewing gum composition outside of the claimed range. Thus, the final chewing gum composition has a moisture content of up to approximately 0.3% by weight without any processing steps directed to drying or removing of moisture. The term substantially moisture free refers to this moisture content.

Chewing gum products made by the process of the present invention have remained fresh, soft and pliable for one year or more with a minimum of protective packaging. For example, unwrapped sticks of chewing gum have remained soft, pliable and have retained their quality and freshness for a year of more in

the open air or in unsealed pouches. This advantage is attributable to the combination of chewing gum ingredients processed in the manner described.

As mentioned above, surprisingly it has been found that a substantially moisture free chewing gum composition, as described for example in EP-A-0176237, allows water insoluble, particulate medicaments to be released in measured essentially reproducible, doses. This is true even though the water insoluble medicament typically may be identical to the filler material used in gum bases which does not release from the bolus upon mastication. This is accomplished by co-spray drying the medicament with sugar in an approximate 50:50 ratio. While these percentages may vary between 40% medicament, e.g. calcium carbonate, to 60% sugar, and 70% medicament, e.g calcium carbonate, to 30% sugar, grainy mouthfeel due to excess medicament, e.g. calcium carbonate, becomes a problem at the higher levels of addition, and co-spray drying becomes difficult as the level of sugar increases.

While other insoluble, particulate medicaments may be co-spray dried along with sugar according to the teachings of this invention, the gum delivery system has been found particularly effective for the delivery of antacids. There are several reasons for this. First, desired dosage levels correlate well with the amount of antacid which can be liberated upon mastication, as will be explained more fully below. Second, dosage of antacid need not be as precise as, for instance, highly potent antibiotics. Third, co-spray drying with sugar, particularly at the 50:50 weight ratio, masks the undesirable gritty mouthfeel associated with these particulate medicaments.

Examples of antacids suitable for use with this invention are magnesium hydroxide, calcium carbonate, and calcium hydroxide. As is apparent the teachings of this invention also provide a delivery system for calcium per se in the form of insoluble calcium salts. The need for supplemental calcium has recently been discovered in mature and older women particularly after they reach menopause. Calcium is used to treat osteoporosis.

The term sugar as used in this disclosure is used interchangeably with water soluble sweetening agents which are susceptible to co-spray drying. Representative illustrations are: water-soluble sweetening agents such as monosaccharides, disaccharides and polysaccharides such as xylose, ribose, mannose, galactose, fructose, dextrose, sucrose, sugar maltose, partially hydrogenized starch or corn syrup solids and sugar alcohols such as sorbitol, xylitol, mannitol and mixtures thereof.

Water soluble sweetening agents are generally present between 25 to 75% by weight of the gum piece. The proportion of the co-spray dried water soluble sweetening agent is employed as a percentage of the total sweetening agent content is determined by the dosage of the medicament desired and the ultimate sweetness level. Interestingly, where glycerol is used instead of corn syrup, added dried sugar can be utilized in addition to comparatively high levels of sweeteners without making a product which is too sweet to be palatable.

Spray drying and co-spray drying are common processes used throughout the food industry. Spray drying, in fact, is commonly used to produce dextrose particles from corn syrup and the inclusion of insoluble medicament particles within this process is contemplated.

Other components traditionally associated with gum manufacture may also be used for the medicament gum of the present invention.

Of course flavouring agents well known in the chewing gum art may be added to the gum compositions including synthetic flavour oils, flavouring and/or oils derived from plants, leaves, flowers and fruits as well as combinations thereof. Representative flavour oils include: spearmint oil, cinnamon oil, oil of wintergreen (methylsalicylate) and peppermint oils as well as their flavouring replacements. Also useful are artificial, natural or synthetic fruit flavours such as citrus oil including lemon, orange, grape, lime and grapefruit and fruit essences including apple, strawberry, cherry, pineapple, banana, etc. Other fruit flavours well known to the art are also employable. The amount of flavouring agent is naturally a matter of preference subject to such factors as flavour type, base type and strength desired. In general, amounts of 0.05% to 3.0% by weight of the final chewing gum composition are useable with amounts in the lower ranges being preferred, with the amount chosen being somewhat dependent upon the particular medicament to be delivered. Flavour masking may be desirable, depending upon the medicament, and, of course, the flavour should neither be of a type nor in a quantity to interfere with the delivery mechanism.

The chewing gum compositions may also contain sweetening agents independent of the spray dried delivery system. Sweetening agent may be selected from a wide range of materials such as water soluble sweetening agents, water soluble artificial sweetening agents and dipeptide-base sweeteners, including mixtures thereof. Without being limited to particular sweeteners, representative tillustrations encompass:

A. Water-soluble sweetening agents such as monosaccharides, disaccharides and polysaccharides such as xylose, ribose, glycose, mannose, galactose, fructose, dextrose, sucrose, sugar maltose, partially hydrogenated starch or corn syrup solids and sugar alcohols such as sorbitol, zylitol, mannitol and

mixtures thereof.

B. Water-soluble artificial sweetener such as the soluble saccharin salts, i.e., sodium or calcium saccharide salts, cyclamate salts, acesulfame-K and the free acid form of saccharin.

C. Dipeptide-base sweetener such as L-aspartyl-L-phenyalylene, methyl ester and materials described in U.S. Patent No. 3,492,131.

The water soluble sweeteners described in category A above are preferably used in amounts of 25% to 75% by weight and most preferably from 50% to 65% by weight of the final chewing gum composition. This percentage includes that which is co-spray dried with the medicaments of the present invention. In contrast, the artificial sweeteners described in categories B and C are used in amounts of 0.005% to 5% and most preferably 0.05% to 2.5% by weight of the final chewing gum composition. These amounts are ordinarily necessary to achieve the desired level of sweetness independent of the flavour level obtained from flavour oils.

The chewing gum compositions of this invention may additionally include conventional additives of colouring agents such as titanium dioxide, additional fillers such as aluminum hydroxide, alumina, aluminum silicates; gum base fillers such as talc and calcium carbonate and combinations thereof; and additional emulsifiers such as glycerol monostearate. The amount of filler in the gum base should remain within the range of 10 to 25% by weight of the gum base.

The invention may be more readily understood by reference to the comparative example set forth below in which the addition of calcium carbonate with and without being co-spray dried with sugar is compared by measuring liberation of the antacid drug through ACP assay.

ACP stands for acid consuming power and is the number of milliliters of 0.1NHCl neutralized. As an example, an ACP value of 50 means that 50 ml of 0.1NHCl have been neutralized. ACP measurement is performed by the following procedure. One piece of gum is heated in a crucible at temperatures of at least 550° C but less than 600° C for a period of between 4 and 12 hours to turn the gum sample to ash.

The ash sample is then dissolved slowly in 30.0 ml of 1.0NHCl after 15 minutes the solution is diluted with water to produce a solution of 100 ml or less. Dilution is done to aid sample transfer. This solution is stirred for 15 minutes and then titrated with standardized 1.0NNaOH to a pH 3.5 end point.

ACP is then calculated according to the following formula:

$$ACP/gm = \frac{\text{Vol (Acid) x Normality (Acid) - Vol (Base) x Normal (Base)}}{\text{Sample wt (gm)}}$$

The table below compares the effect of the addition of co-spray dried sucrose and calcium carbonate to a conventional gum formula i.e. one having a conventional moisture content, (comparative Sample 1) with similar additions of that co-spray dried mixture to a substantially moisture free gum base (Inventive Samples 2 and 6). Comparative Samples 3 and 4 compare the addition of a mixture of granulated sucrose and granulated calcium carbonate in varying ratios to a substantially moisture free gum with the medicament containing gum of this invention (Inventive Samples 2 and 6). Comparative sample 5 compares the effect of the addition of calcium carbonate only to a substantially moisture free gum base.

Samples 2 through 6 were prepared using the process described above, and Sample 1 was prepared according to conventional process techniques as described above with reference to the prior art. All numbers given below are by weight %.

TABLE

| | Comparative | Inventive | Comparative | | | Inventive |
|---|---|---|---|---|---|---|
| Sample | 1 | 2 | 3 | 4 | 5 | 6 |
| 1. Gum base | | | | | | |
| A. Gum Base | 20.00 | 22.00 | 22.00 | 22.00 | 22.00 | 22.00 |
| 2 Sweeteners | | | | | | |
| A. Sucrose | 25.7875 | 30.7375 | 22.80 | 43.80 | 71.25 | 15.025 |
| B. Corn syrup | 16.5 | | | | | |
| C. Micro flavor Buds | -- | 4.00 | 4.00 | 4.00 | 4.00 | 4.00 |
| 3. Flavors | | | | | | |
| A. Liquid flavor | 1.00 | 1.00 | 1.20 | 1.20 | 1.20 | 1.20 |
| B. Spray dried flavor | .50 | .50 | .50 | .50 | .50 | .50 |
| C. Acids | -- | -- | -- | -- | -- | -- |
| 4. Softeners | | | | | | |
| A. Glycerin | .45 | 5.00 | 5.00 | 5.00 | 5.00 | 5.00 |
| B. Lecithin | .50 | .50 | .50 | .50 | .50 | .50 |
| C. Color | .075 | .075 | .075 | .075 | .075 | .075 |
| 5. A. 50-50 (Sucrose - $CaCO_3$) Agglomeration | 35.187 | 35.187 | -- | -- | -- | 52.6 |
| B. 60-40 Granulation addition | -- | -- | 44.00 | -- | -- | -- |
| C. 75-25 $CaCO_3$ - sugar granulation | -- | -- | -- | 23.00 | -- | -- |
| Add ACP to Product | 119.4 | 119.4 | 99.4 | 85.40 | 106 | 106 |
| Release ACP | 26.5 | 47.05 | Trace | Trace | 4.4 | 76.20 |

After each sample gum stick was made it was chewed for 20 minutes and the calcium carbonate remaining in the bolus was measured. This amount was subtracted from the total added to the sample and the difference, i.e, the amount liberated during mastication, was used to determine the ACP value available to neutralize excess stomach acidity. This value was compared to the total ACP value which would be available if all that was added to the gum stick was liberated. It was noted that only four samples, i.e , 1, 2, 5 and 6 showed any measurable ACP release, the lowest of this group being the sample (Sample 5) which had direct calcium addition to the substantially moisture free gum base. Inventive Sample 2 shows almost twice the amount of ACP released than that of Comparative Sample 1. Further experiments have indicated substantial variance in ACP value for the conventional gum of sample 1. Furthermore, when Inventive Sample 2 and Comparative Sample 4 are compared, an increase in ACP released roughly proportional to the increase in calcium carbonate added was observed. The increased amount of the co-spray dried mixture used in Inventive Sample 6 compared to that in Inventive Sample 2 also correlated to a decrease in sucrose addition to the gum formula. It is apparent that calcium carbonate and similar medicament levels can be easily manipulated by adjusting these two levels.

It is generally accepted that to be classified as an antacid approximately a 50 ACP value is necessary. This value is both obtainable and reproducible according to the teachings of this invention.

While not wishing to be bound by any theory it is believed that the consistant, reproducibly high levels

of antacid liberation is due to the ease of liberation of the co-spray dried sweetening agent and medicament agglomerate upon mastication. The sugar-antacid agglomerate apparently releases readily from the anhydrous gum of this invention as sugar does from all gums during mastication.

## Claims

### Claims for the following Contracting States : BE, FR, DE, SE, CH, GB

1. A medicament-containing chewing gum composition having improved delivery properties and a moisture content up to 0.3% by weight of the final composition which composition comprises:
   (i) a gum base, which softens in the temperature range of 40°C to 60°C;
   (ii) a softener; and
   (iii) a co-spray dried mixture of a sweetening agent and a water-insoluble, particulate medicament, and wherein the moisture content of the chewing gum composition is at equilibrium in a relative humidity in the range 15 to 30% at a temperature of 23°C.

2. A chewing gum composition according to claim 1, wherein the medicament is an antacid.

3. A chewing gum composition according to claim 2, wherein the antacid is magnesium hydroxide, calcium carbonate or aluminum hydroxide.

4. A chewing gum composition according to claim 2 or 3, wherein the antacid liberated during mastication has an Acid Consuming Power (ACP) value of at least 50.

5. A chewing gum composition according to any preceding claim, wherein the medicament is present in an amount of 40 to 70% by weight of the co-spray dried mixture.

6. A chewing gum composition according to claim 5, wherein the medicament is present in an amount of 50% by weight of the co-spray dried mixture.

7. A chewing gum composition according to any preceding claim, wherein the sweetening agent is sucrose.

8. A chewing gum composition according to any preceding claim, wherein the medicament is calcium carbonate.

9. A chewing gum composition according to any preceding claim, wherein the moisture content is at equilibrium in a relative humidity in the range of 21 to 25% at a temperature of 23°C.

10. A process for the preparation of a chewing gum composition according to any one of claims 1 to 9, which process comprises the steps of:
    (a) softening the gum base using a temperature in the range of 40°C to 60°C with the addition of a softener;
    (b) adding a co-spray dried mixture of a sweetening agent and a medicament;
    (c) adding any optional ingredients such as a flavouring agent or texturizing agent, e.g. a glycol; continuing to combine until a homogeneous, pliable composition is obtained;
    (d) extruding the composition;
    (e) forming the composition into chewing gum pieces without cooling; and wrapping the chewing gum without prior conditioning.

### Claims for the following Contracting States : GR, ES

1. A process for the preparation of a medicament-containing chewing gum composition having improved delivery properties and a moisture content up to 0.3% by weight of the final composition which composition comprises:
   (i) a gum base which softens in a temperature range of 40°C to 60°C.
   (ii) a softener; and
   (iii) a co-spray dried mixture of a sweetening agent and a water-insoluble, particulate medicament, and wherein the moisture content of the chewing gum composition is at equilibrium in a relative

humidity in the range 15 to 30% at a temperature of 23 °C, which process comprises the step of:

(a) softening the gum base using a temperature in the range of 40 °C to 60 °C with the addition of a softener;

(b) adding a co-spray dried mixture of a sweetening agent and a medicament;

(c) adding any optional ingredients such as a flavouring agent or a texturizing agent, e.g., glycol; continuing to combine until a homogeneous, pliable composition is obtained;

(d) extruding the composition;

(e) forming the composition into suitable chewing gum pieces without cooling; and wrapping the chewing gum without prior conditioning.

**2.** A process according to claim 1, wherein the medicament is an antacid.

**3.** A process according to claim 2, wherein the antacid is magnesium hydroxide, calcium carbonate or aluminum hydroxide.

**4.** A process according to claim 2 or 3, wherein the antacid liberated during mastication has an Acid Consuming Power (ACP) value of at least 50.

**5.** A process according to any preceding claim, wherein the medicament is present in an amount of 40 to 70% by weight of the co-spray dried mixture.

**6.** A process according to claim 5, wherein the medicament is present in an amount of 50% by weight of the co-spray dried mixture.

**7.** A process according to any preceding claim, wherein the sweetening agent is sucrose.

**8.** A process according to any preceding claim, wherein the medicament is calcium carbonate.

**9.** A process according to any preceding claim, wherein the moisture content is at equilibrium in a relative humidity in the range of 21 to 25% at a temperature of 23 °C

**Revendications**

**Revendications pour les Etats contractants suivants : BE, FR, DE, SE, CH, GB**

**1.** Composition de chewing-gum contenant un médicament, ayant des propriétés améliorées de libération du médicament et une teneur en humidité allant jusqu'à 0,3% en poids de la composition finale, laquelle composition comprend :

(i) une gomme base, qui se ramollit dans la gamme des températures de 40 °C à 60 °C;

(ii) un assouplissant; et

(iii) un mélange séché par co-pulvérisation d'un agent édulcorant et d'un médicament particulaire insoluble dans l'eau, où la teneur en humidité de la composition de chewing-gum est à l'équilibre dans une humidité relative dans la gamme de 15 à 30%, à une température de 23 °C.

**2.** Composition de chewing-gum selon la revendication 1, où le médicament est un antiacide.

**3.** Composition de chewing-gum selon la revendication 2, où l'antiacide est de l'hydroxyde de magnésium, du carbonate de calcium ou de l'hydroxyde d'aluminium.

**4.** Composition de chewing-gum selon la revendication 2 ou 3, où l'antiacide libéré durant la mastication a une capacité de neutralisation d'acide (CNA) d'au moins 50.

**5.** Composition de chewing-gum selon l'une quelconque des revendications précédentes, où le médicament est présent en une proportion allant de 40 à 70% en poids du mélange séché par co-pulvérisation.

**6.** Composition de chewing-gum selon la revendication 5, où le médicament est présent en une proportion de 50% en poids du mélange séché par co-pulvérisation.

7. Composition de chewing-gum selon l'une quelconque des revendications précédentes, où l'agent édulcorant est le saccharose.

8. Composition de chewing-gum selon l'une quelconque des revendications précédentes, où le médicament est le carbonate de calcium.

9. Composition de chewing-gum selon l'une quelconque des revendications précédentes, où la teneur en humidité est à l'équilibre dans une humidité relative dans la gamme de 21 à 25% à la température de 23° C.

10. Procédé de préparation d'une composition de chewing-gum selon l'une quelconque des revendications 1 à 9, lequel procédé comprend les étapes consistant à : (a)ramollir la gomme base en utilisant une température dans la gamme de 40° C à 60° C, tout en ajoutant un assouplissant;
    (b) ajouter un mélange séché par co-pulvérisation, constitué d'un agent édulcorant et d'un médicament;
    (c) ajouter des ingrédients facultatifs tels qu'un agent aromatisant ou un agent de texturation du type glycol; continuer de mélanger jusqu'à l'obtention d'une composition homogène et souple;
    (d) extruder la composition;
    (e) former la composition de chewing-gum en pièces, sans la refroidir; et envelopper sans autre conditionnement préalable du chewing-gum.

**Revendications pour les Etats contractants suivants : GR, ES**

1. Procédé de préparation d'une composition de chewing-gum contenant un médicament, ayant des propriétés améliorées de libération du médicament et une teneur en humidité allant jusqu'à 0,3% en poids de la composition finale, laquelle composition comprend :
    (i) une gomme base, qui se ramollit dans la gamme des températures de 40° C à 60° C;
    (ii) un assouplissant; et
    (iii) un mélange séché par co-pulvérisation d'un agent édulcorant et d'un médicament particulaire insoluble dans l'eau, où la teneur en humidité de la composition de chewing-gum est à l'équilibre dans une humidité relative dans la gamme de 15 à 30%, à une température de 23° C, lequel procédé comprend les étapes consistant à :
        (a) ramollir la gomme base en utilisant une température dans la gamme de 40° C à 60° C, tout en ajoutant un assouplissant
        (b) ajouter un mélange séché par co-pulvérisation, constitué d'un agent édulcorant et d'un médicament;
        (c) ajouter des ingrédients facultatifs tels qu'un agent aromatisant ou un agent de texturation du type glycol; continuer de mélanger jusqu'à l'obtention d'une composition homogène et souple;
        (d) extruder la composition;
        (e) former la composition de chewing-gum en pièces appropriées, sans la refroidir; et envelopper sans autre conditionnement préalable du chewing-gum.

2. Procédé selon la revendication 1, où le médicament est un antiacide.

3. Procédé selon la revendication 2, où l'antiacide est de l'hydroxyde de magnésium, du carbonate de calcium ou de l'hydroxyde d'aluminium.

4. Procédé selon la revendication 2 ou 3, où l'antiacide libéré durant la mastication a une capacité de neutralisation d'acide (CNA) d'au moins 50.

5. Procédé selon l'une quelconque des revendications précédentes, où le médicament est présent en une proportion allant de 40 à 70% en poids du mélange séché par co-pulvérisation.

6. Procédé selon la revendication 5, où le médicament est présent en une proportion de 50% en poids du mélange séché par co-pulvérisation.

7. Procédé selon l'une quelconque des revendications précédentes, où l'agent édulcorant est le saccharose.

8. Procédé selon l'une quelconque des revendications précédentes, où le médicament est le carbonate de calcium.

9. Procédé selon l'une quelconque des revendications précédentes, où la teneur en humidité est à l'équilibre dans une humidité relative dans la gamme de 21 à 25%, à la température de 23° C.

**Patentansprüche**
**Patentansprüche für folgende Vertragsstaaten : BE, FR, DE, SE, CH, GB**

1. Eine, ein Medikament enthaltende Kaugummimischung mit verbesserten Abgabeeigenschaften und einem Feuchtigkeitsgehalt bis zu 0,3 Gewichtsprozenten der Endmischung, wobei das Gemisch enthält:
   (i) eine Gummibasis, die im Temperaturbereich von 40 bis 60° C weich wird;
   (ii) einen Weichmacher; und
   (ii) ein kospraygetrocknetes Gemisch eines Süssstoffes und eines wasserunlöslichen Partikelmedikamentes, wobei der Feuchtigkeitsgehalt der Kaugummimischung bei einer relativen Feuchtigkeit von 15 bis 30% und einer Temperatur von 23° C im Gleichgewicht ist.

2. Eine Kaugummimischung nach Patentanspruch 1, worin das Medikament ein Antazidum ist.

3. Eine Kaugummimischung nach Patentanspruch 2, worin das Antazidum ein Magnesiumhydroxid, Kalziumkarbonat oder Aluminiumhydroxid ist.

4. Eine Kaugummimischung nach Patentanspruch 2 oder 3, worin das während der Mastikation freigesetzte Antazidum einen ACP-Wert (Säureaufnahmevermögenswert) von mindestens 50 hat.

5. Eine Kaugummimischung nach einem der vorhergehenden Patentansprüche, worin das Medikament in einer Menge von 40 bis 70 Gewichtsprozenten des kospraygetrockneten Gemisches vorhanden ist.

6. Eine Kaugummimischung nach Patentanspruch 5, worin das Medikament in einer Menge von 50 Gewichtsprozenten des kospraygetrockneten Gemisches vorhanden ist.

7. Eine Kaugummimischung nach einem der vorhergehenden Patentansprüche, worin der Süssstoff Sukrose ist.

8. Eine Kaugummimischung nach einem der vorhergehenden Patentansprüche, worin das Medikament Kalziumkarbonat ist.

9. Eine Kaugummimischung nach einem der vorhergehenden Patentansprüche, bei der Feuchtigkeitsgehalt der Kaugummimischung bei einer relativen Feuchtigkeit von 21 bis 25% und einer Temperatur von 23° C im Gleichgewicht ist.

10. Ein Verfahren zur Herstellung einer Kaugummimischung nach einem der Patentansprüche 1 bis 9, wobei das Verfahren aus folgenden Schritten besteht:
   (a) Erweichen der Gummibasis in einem Temperaturbereich von 40 bis 60° C unter Zusatz eines Weichmachers;
   (b) Zugabe eines kospraygetrockneten Gemisches aus einem Süssstoff und einem Medikament;
   (c) Zugabe beliebig ausgewählter Ingredienzen, wie Geschmackstoffen oder Texturiermitteln, beispielsweise Glykol; Fortsetzen des Verbindens bis eine homogene, biegsame Mischung erhalten wird;
   (d) Extrudieren der Mischung;
   (e) Formen der Mischung zu Kaugummistücken ohne Kühlung; und Verpacken des Kaugummis ohne vorheriges Konditionieren.

**Patentansprüche für folgende Vertragsstaaten : GR, ES**

1. Ein Verfahren zur Herstellung einer ein Medikament enthaltende Kaugummimischung mit verbesserten Abgabeeigenschaften und einem Feuchtigkeitsgehalt bis zu 0,3 Gewichtsprozenten der Endmischung, wobei das Gemisch enthält:

(i) eine Gummibasis, die im Temperaturbereich von 40 bis 60 °C weich wird;
(ii) einen Weichmacher; und
(ii) ein kospraygetrocknetes Gemisch eines Süssstoffes und eines wasserunlöslichen Partikelmedikamentes, wobei der Feuchtigkeitsgehalt der Kaugummimischung bei einer relativen Feuchtigkeit von 15 bis 30% und einer Temperatur von 23 °C im Gleichgewicht ist, und wobei das Verfahren aus folgenden Schritten besteht:
(a) Erweichen der Gummibasis in einem Temperaturbereich von 40 bis 60 °C unter Zusatz eines Weichmachers;
(b) Zugabe eines kospraygetrickneten Gemisches aus einem Süssstoff und einem Medikament;
(c) Zugabe beliebig ausgewählter Ingredienzen, wie Geschmackstoffen oder Texturiermitteln, beispielsweise Glykol; Fortsetzen des Verbindens bis eine homogene, biegsame Mischung erhalten wird;
(d) Extrudieren der Mischung;
(e) Formen der Mischung zu Kaugummistücken ohne Kühlung; und Verpacken des Kaugummis ohne vorheriges Konditionieren.

**2.** Ein Verfahren nach Patentanspruch 1, worin das Medikament ein Antazidum ist.

**3.** Ein Verfahren nach Patentanspruch 2, worin das Antazidum ein Magnesiumhydroxid, Kalziumkarbonat oder Aluminiumhydroxid ist.

**4.** Ein Verfahren nach Patentanspruch 2 oder 3, worin das während der Mastikation freigeseetzte Antazidum einen ACP-Wert (Säureaufnahmevermögenswert) von mindestens 50 hat.

**5.** Ein Verfahren nach einem der vorhergehenden Patentansprüche, worin das Medikament in einer Menge von 40 bis 70 Gewichtsprozenten des kospraygetrockneten Gemisches vorhanden ist.

**6.** Ein Verfahren nach Patentanspruch 5, worin das Medikament in einer Menge von 50 Gewichtsprozenten des kospraygetrockneten Gemisches vorhanden ist.

**7.** Ein Verfahren nach einem der vorhergehenden Patentansprüche, worin der Süssstoff Sukrose ist.

**8.** Ein Verfahren nach einem der vorhergehenden Patentansprüche, worin das Medikament Kalziumkarbonat ist.

**9.** Ein Verfahren nach einem der vorhergehenden Patentansprüche, bei dem der Feuchtigkeitsgehalt der Kaugummimischung bei einer relativen Feuchtigkeit von 21 bis 25% und einer Temperatur von 23 °C im Gleichgewicht ist.